# DEMANDE DE BREVET EUROPEEN

(11) **EP 0 532 401 A1**
(43) Date de publication de la demande: **17.03.1993**
(21) Numéro de dépôt: 92402444.1
(22) Date de dépôt: 07.09.1992
(51) Int. Cl.: C07C 51/487

(54) **Procédé de traitement d'une solution contenant de l'acide acétique en présence d'halogènes**

(30) Priorité: 09.09.1991 FR 9111103
(71) Demandeur: COMMISSARIAT A L'ENERGIE ATOMIQUE, F-75015 Paris (FR)
(72) Inventeur: Morin, Michel, F-84800 l'Isle sur la Sorgue (FR); Lanoe, Jean-Yves, F-30200 Bagnols/Ceze (FR); Ros, Pierre, Sauveterre, F-30150 Roquemaure (FR)
(74) Mandataire: Dubois-Chabert, Guy

(57) **Abrégé**

L'invention concerne un procédé de traitement d'une solution contenant de l'acide acétique en présence d'halogènes.

Le but de l'invention est de réaliser un procédé permettant de diminuer la demande chimique en oxygène de cette solution.

Ce but est atteint à l'aide d'un procédé consistant à :
a) traiter cette solution par une solution (10) permettant de réduire les halogènes en halogénures,
b) mettre, dans une colonne d'extraction (2), la solution à traiter en contact avec un solvant organique (8) d'extraction de l'acide acétique contenant essentiellement un trialkylphosphate et un hydrocarbure, et
c) mettre, dans une colonne de réextraction (4), le solvant (14) chargé en acide acétique, en contact avec une solution basique (16) capable de neutraliser le solvant et de réextraire l'acétate de sodium (20) formé.

Ce procédé est plus particulièrement destiné à être utilisé dans l'industrie de la synthèse organique.

## Description

La présente invention concerne un procédé de traitement d'une solution contenant de l'acide acétique, en présence d'halogènes.

Les procédés de synthèse organique que l'on trouve par exemple dans l'industrie de la papeterie, de la production d'herbicides ou de fongicides conduisent fréquemment à la formation d'effluents contenant de grandes quantités d'acide acétique, en présence d'halogènes et éventuellement de sulfates ou de sodium. Ces effluents présentent une demande chimique en oxygène (D.C.O) élevée. La D.C.O. correspond à la quantité d'oxygène nécessaire à une dégradation chimique de ces effluents. Or, pour respecter les normes antipollution en vigueur, on ne peut rejeter dans l'environnement d'effluents présentant une trop grande D.C.O.

La quasi totalité de la D.C.O étant due principalement à l'acide acétique, il est nécessaire de séparer l'acide acétique du reste des effluents qui poseront alors moins de problèmes de rejet dans l'environnement.

Les procédés actuellement connus pour extraire l'acide acétique d'une solution sont par exemple des procédés d'extraction liquide-liquide. Ceux-ci consistent à mettre en contact la solution à traiter avec un solvant organique tel que ceux de la famille des organophosphorés, des amines tertiaires et des esters, comme par exemple l'acétate d'éthyle. Ces contacts liquide-liquide sont réalisés de préférence à contre courant dans des extracteurs qui peuvent être avantageusement du type colonne pulsée.

Or, la présence d'halogénes dans un effluent acide ne permet pas d'utiliser ces solvants organiques classiques car la sélectivité de ceux-ci est mauvaise et il n'est donc pas possible de séparer correctement l'acide acétique des composés halogénés. En outre, au cours de l'extraction, le potentiel red-ox de la phase aqueuse augmente, en libérant ainsi les halogènes sous leur forme moléculaire qui dégrade l'extractant et le diluant. Enfin, la présence d'halogénes dans le solvant chargé en acide acétique pose de gros problèmes de corrosion lors de l'étape de distillation permettant de recycler le solvant pour le renvoyer dans la colonne d'extraction.

On connaît déjà d'après le document US-A-4 143 066 un procédé de séparation et d'élimination d'un acide carboxylique inférieur (l'acide acétique, par exemple) d'un mélange comprenant un solvant organique, un agent d'extraction, de l'eau et cet acide carboxylique. Ce document précise que l'on peut utiliser un trialkylphosphate comme agent d'extraction et un hydrocarbure comme solvant organique. Toutefois, ce document ne préconise aucune solution d'extraction dans le cas où des halogènes seraient également présents dans ce mélange.

On connaît également dans l'art antérieur (Journal of Chemical and Engineering Data, Vol.23, n°2, 1978, p.144) un procédé d'extraction de l'acide acétique utilisant du tributylphosphate (TBP) dilué par un hydrocarbure. Toutefois, ces extractions par solvant sont réalisées sur des solutions d'acide acétique contenant des impuretés en faibles quantités. De plus, ces techniques ont pour but d'extraire l'acide acétique sous forme moléculaire et de traiter ensuite la solution obtenue par distillation pour récupérer cet acide. Dans le cas des effluents d'acide acétique de la synthèse organique, la présence simultanée d'halogénes amène des problèmes supplémentaires de corrosion et de formation de nouveaux produits avec le solvant, ces nouveaux produits n'étant pas extractibles.

De plus, dans le cas présent on ne cherche pas à produire de l'acide acétique mais seulement à éliminer celui-ci, sous quelque forme que ce soit, afin de permettre le rejet de l'effluent dans l'environnement selon les normes en vigueur.

L'invention a justement pour objet de réaliser un procédé permettant d'éliminer l'acide acétique d'un effluent contenant simultanément de l'acide acétique et des halogènes, afin de diminuer la demande chimique en oxygène de cet effluent.

De façon plus précise, l'invention concerne un procédé de traitement d'une solution contenant de l'acide acétique, en présence d'halogénes, consistant à :
a) traiter cette solution par une solution permettant de réduire les halogénes en halogénures,
b) mettre, dans une colonne d'extraction, la solution à traiter en contact avec un solvant organique d'extraction de l'acide acétique contenant essentiellement un trialkylphosphate et un hydrocarbure, et
c) mettre, dans une colonne de réextraction, le solvant chargé en acide acétique en contact avec une solution basique capable de neutraliser le solvant et de réextraire l'acétate de sodium formé.

L'acétate de sodium est extrait de préférence sous forme cristallisée.

Lorsque l'on souhaite utiliser de façon noble l'acétate de sodium qui a été produit par le procédé décrit ci-dessus, on peut effectuer une étape supplémentaire de lavage. A cet effet, l'invention consiste également après avoir procédé à la première extraction, à :
- laver, dans une colonne de lavage, le solvant chargé d'acide acétique, avec une solution aqueuse de lavage,
- réinjecter dans la colonne d'extraction, la solution de lavage sortant de la colonne de lavage, et
- mettre, dans une colonne de réextraction, le solvant chargé en acide acétique et lavé, en contact avec une solution alcaline capable de neutraliser ce solvant et de réextraire l'acétate de sodium formé.

Cette étape de lavage permet essentiellement de faire refluer vers l'extraction les impuretés, c'est-à-dire les halogénures. On pourra ainsi facilement récupérer à la fin du procédé un acétate de sodium titrant 99 % de pureté. Cet acétate de sodium peut également être produit sous forme cristallisée en refroidissant la sortie de l'appareil de réextraction.

Pour permettre l'extraction de l'acide acétique en utilisant un solvant organique à base de trialkylphosphate et d'hydrocarbure, il est nécessaire que l'effluent soit traité au préalable avec une solution aqueuse de métabisulfite de sodium afin de réduire les halogènes en halogénures. On préfère utiliser le métabisulfite de sodium en raison de son faible coût et de sa forme oxydée, après réaction, non gênante pour le procédé.

Le trialkylphosphate généralement utilisé est le tri-n-butylphosphate (TBP) ou le triisobutylphosphate (TIBP) du fait de leur bon pouvoir d'extraction.

L'hydrocarbure utilisé est en général le tétrapropyléne hydrogéné (TPH).

Au cours de diverses étapes du procédé, on souhaite éviter la formation d'émulsions difficiles à décanter et qui nuiraient au fontionnement hydrodynamique des colonnes pulsées. Pour y parvenir, il convient de veiller à ce que la phase organique soit plus légère que la phase aqueuse. C'est la raison pour laquelle on doit diluer le trialkylphosphate pur (densité de 0,97 pour le TBP par exemple) par un hydrocarbure liquide moins dense (densité de 0,76 pour le TPH par exemple). Toutefois, il est important de ne pas trop diluer le trialkylphosphate afin de ne pas avoir à augmenter la longueur des colonnes d'extraction ou à utiliser un rapport de débits (phase organique/phase aqueuse) pénalisant.

Dans la pratique, on peut diluer le trialkylphosphate jusqu'à ce qu'il représente 65 à 85 % du volume du solvant, mais de préférence on utilisera 80 % de TBP pour 20 % de TPH.

On diminue ainsi la densité et la viscosité du solvant organique tout en augmentant sa tension interfaciale avec les solutions aqueuses en contact. Il n'est alors plus nécessaire d'opérer à chaud pour avoir un bon fonctionnement hydraulique des contacts d'extraction (colonne pulsée). Ainsi, l'extraction peut avoir lieu à température ambiante (environ 25°C), ce qui réduit son coût.

L'étape de lavage permettant de débarrasser le solvant des impuretés telles que les halogénures peut être réalisée avec de l'eau déminéralisée, ou avec une solution aqueuse de métabisulfite de sodium à une concentration de 100 à 500 mg/l.

Enfin, dans la dernière étape d'extraction, on utilise comme solution aqueuse une solution de soude permettant de neutraliser le solvant, c'est-à-dire de former l'acétate de sodium et de neutraliser les produits de dégradation du tributylphosphate tel que l'acide dibutylphosphate (HDBP) ou l'acide monobutylphosphate (H₂MBP). Le solvant peut alors être recyclé vers la première colonne d'extraction.

Le procédé selon l'invention peut être mis en oeuvre de façon simple, dans des installations industrielles existantes.

D'autres caractéristiques et avantages de l'invention ressortiront mieux à la lecture de la description qui va suivre, donnée à titre illustratif et non limitatif, et faite en référence aux dessins annexés dans lesquels :
- la figure 1 représente schématiquement un premier mode de réalisation du dispositif de mise en oeuvre du procédé conforme à l'invention, et
- la figure 2 représente un second mode de réalisation du dispositif de la figure 1.

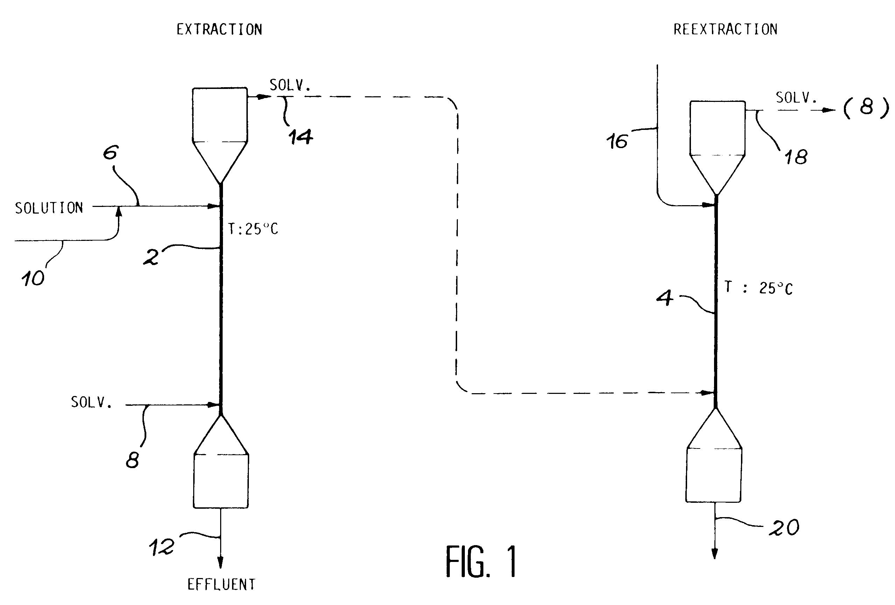

Comme illustré en figure 1, selon le premier mode de réalisation, le dispositif comporte deux appareils 2, 4 d'extraction liquide-liquide du type colonne pulsée, destinés respectivement à l'extraction de l'acide acétique et la réextraction de l'acétate de sodium.

La solution à traiter est introduite en 6 en tête de la colonne d'extraction 2 dans laquelle l'acide acétique est extrait, à contre-courant du solvant organique d'extraction introduit à la base du fût de la colonne 2 au point 8.

Toutefois, dans la mesure où cette extraction n'est possible que si les halogènes sont sous forme réduite, on introduit au préalable dans la solution à traiter la solution aqueuse de métabisulfite de sodium 10.

Le mélange de solvants introduit en 8 contient de préférence 80 % en volume de TBP et 20 % en volume de TPH pour les raisons évoquées précédemment.

Dans ces conditions, l'opération d'extraction de l'acide acétique par le TBP peut être conduite à la température ambiante, de l'ordre de 25°C. L'effluent sortant en 12, au pied de la colonne d'extraction 2, contient essentiellement des halogénures à éliminer et présente une D.C.O très faible, qui peut d'ailleurs être encore diminuée par un stripage de l'effluent à la vapeur (terminologie anglosaxonne : stripping).

Le solvant organique chargé en acide acétique, sortant en 14, en haut de la colonne d'extraction 2 est traité à contre courant dans la colonne de réextraction 4 de l'acétate de sodium, avec une solution 16 de soude 10 M, introduite en tête de colonne. Cette réextraction de l'acétate de sodium est également effectuée à température ambiante (25°C). Dans cette colonne de réextraction 4, le rapport des débits entre la phase organique et la phase aqueuse est de préférence supérieur ou égal à 15. Le solvant déchargé sortant en haut de la colonne en 18 est neutralisé et peut être recyclé tel quel au pied de la colonne d'extraction 2 au point 8. Quant à la solution d'acétate de sodium sortant en 20 de la colonne de réextraction 4, elle peut être soit détruite, soit produite sous forme de cristaux en refroidissant la sortie de l'appareil de réextraction.

On donne ci-après, un exemple de mise en oeuvre du procédé conforme à l'invention.

### Exemple 1

Dans une installation analogue à celle qui vient d'être décrite, on traite un effluent provenant d'un procédé de synthèse organique. La composition de l'effluent à traiter est la suivante :
CH₃COOH = 35 g/l D.C.O = 44,8 g/l d'oxygène
halogènes :
Br⁻ = 8,35 g/l Cl⁻ = 67,7 g/l

L'effluent à traiter et le solvant organique sont introduits dans la colonne d'extraction 2 avec un débit de solvant correspondant à 80 % du débit aqueux (effluent).

Les opérations d'extraction et de réextraction sont réalisées à une température de 25°.

L'effluent obtenu en 12 contient 0,81 g par litre de CH₃COOH, 7,0 g/l de Br⁻, 67,5 g/l de Cl⁻ et 4,0 g/l de D.C.O. Cette technique permet donc de réduire la D.C.O d'un facteur 10.

Le débit du solvant 14 chargé en acide acétique introduit dans la colonne de réextraction 4 est égal à 0,85 fois le débit de l'effluent introduit en 8. La solution produite en 20 a la composition suivante :
acétate de sodium = 400 g/l à pH 6
D.C.O = 302 g/l
Cl⁻ = 7,5 g/l et Br⁻ = 4,3 g/l, soit moins de 2 % d'halogénures.

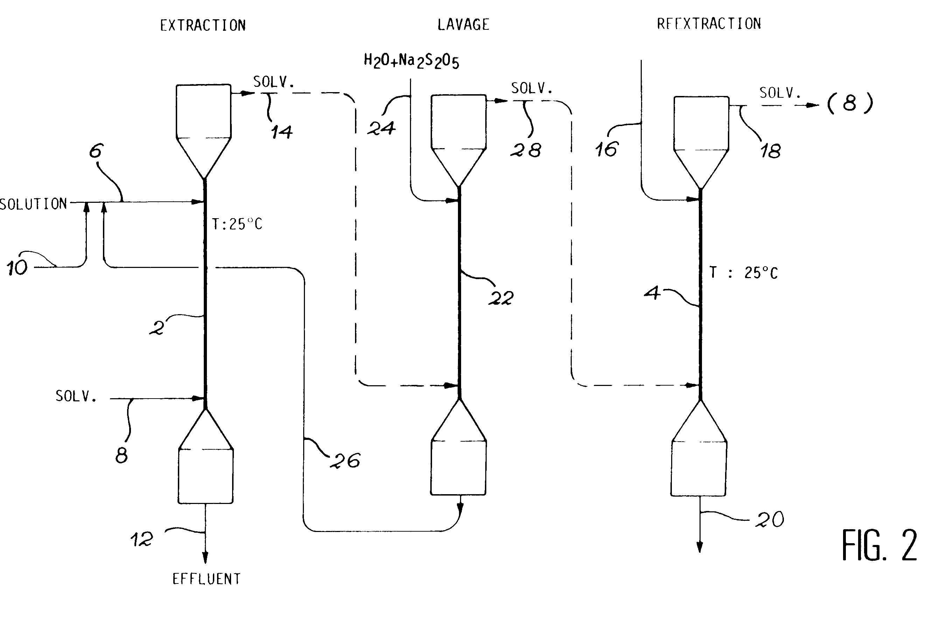

Un second mode de réalisation de l'invention va maintenant être décrit en faisant référence à la figure 2 dans laquelle les éléments identiques à ceux de la figure 1 portent la même référence.

Ce second mode de réalisation comprend une étape supplémentaire de lavage.

Dans ce cas, le solvant organique chargé en acide acétique, sortant en 14 en haut de la colonne d'extraction 2, est traité à contre courant dans une colonne de lavage 22 par une solution aqueuse de métabisulfite de sodium, introduite en 24 en haut de la colonne de lavage 22. Ce lavage est effectué à température ambiante. Le reflux de lavage 26 sortant en pied de la colonne de lavage 22 et qui contient les impuretés telles que les halogénures est renvoyé en 6 sur le fût de la colonne d'extraction 2. Le solvant 28 chargé en acide acétique et lavé, sortant en 28 au sommet de la colonne de lavage 22 est ensuite mis en contact dans la colonne de réextraction 4, à contre courant avec la solution de soude 16 introduite en tête de cette colonne. Le reste des opérations est identique à ce qui a été décrit précédemment pour la figure 1.

On donne ci-après un exemple de mise en oeuvre du procédé conforme au deuxième mode de réalisation de l'invention.

### Exemple 2

La composition de la solution à traiter est identique à celle de l'exemple 1.

Le solvant organique est envoyé en 8 avec un débit identique à celui de la solution à traiter injectée en 6. Le débit du solvant 14 chargé en acide acétique, sortant de la colonne 2 est égal à 1,1 fois le débit de la solution introduite en 6. Par ailleurs, le débit de la solution de lavage introduite en 24 est égal à 0,25 fois le débit de la solution introduite en 6.

Les opérations d'extraction, de lavage et de réextraction sont réalisées à une température de 25°C.

L'effluent obtenu en 12 présente une teneur en acide acétique inférieure à 1 g/l et une DCO de 4,8 g/l. Il peut donc sans problème être relâché dans l'environnement.

La solution produite en 20 a la composition suivante :
Br⁻ = 2 g/l⁻ Cl⁻ = 2,7 g/l
acétate de sodium = 350 à 400 g/l.

Un essai de longue durée de ce procédé (70 heures environ) avec recyclage du solvant a permis de montrer que le solvant et le diluant ne subissaient pas de dégradation significative.

## Revendications

1. Procédé de traitement d'une solution contenant de l'acide acétique, en présence d'halogènes, caractérisé en ce qu'il consiste à :
a) traiter cette solution par une solution (10) permettant de réduire les halogènes en halogénures,
b) mettre, dans une colonne d'extraction (2), la solution à traiter en contact avec un solvant organique (8) d'extraction de l'acide acétique contenant essentiellement un trialkylphosphate et un hydrocarbure, et
c) mettre, dans une colonne de réextraction (4), le solvant (14) chargé en acide acétique, en contact avec une solution basique (16) capable de neutraliser le solvant et de réextraire l'acétate de sodium (20) formé.

2. Procédé de traitement selon la revendication 1, caractérisé en ce qu'il consiste à :
a) traiter cette solution par une solution (10) permettant de réduire les halogènes en halogénures,
b) mettre, dans une colonne d'extraction (2), la solution à traiter en contact avec un solvant organique (8) d'extraction de l'acide acétique contenant essentiellement un trialkylphosphate et un hydrocarbure,
c) laver, dans une colonne de lavage (22), le solvant (14) chargé d'acide acétique, avec une solution aqueuse de lavage (24),
d) réinjecter la solution de lavage (26) sortant de la colonne de lavage (22) dans la colonne d'extraction (2), et
e) mettre dans une colonne de réextraction (4) le solvant (28) chargé en acide acétique et lavé, en contact avec une solution alcaline (16) capable de neutraliser le solvant et de réextraire l'acétate de sodium formé.

3. Procédé de traitement selon la revendication 1 ou 2, caractérisé en ce que la solution (10) permettant de réduire les halogènes en halogénures est une solution aqueuse de métabisulfite de sodium.

4. Procédé de traitement selon la revendication 1 ou 2, caractérisé en ce que le solvant organique (8) contient entre 65 et 85 % de trialkylphosphate et entre 15 et 35 % d'hydrocarbure, en volume.

5. Procédé de traitement selon la revendication 4, caractérisé en ce que le solvant organique (8) contient environ 80 % de trialkylphosphate et environ 20 % d'hydrocarbure, en volume.

6. Procédé de traitement selon l'une des revendications précédentes, caractérisé en ce que le trialkylphosphate est le tri-n-butylphosphate (TBP).

7. Procédé de traitement selon l'une des revendications précédentes, caractérisé en ce que l'hydrocarbure est le tétrapropylène hydrogéné (TPH).

8. Procédé de traitement selon la revendication 2, caractérisé en ce que la solution aqueuse de lavage (24) est une solution de métabisulfite de sodium à une concentration comprise entre 100 et 500 mg/l.

9. Procédé de traitement selon la revendication 2, caractérisé en ce que la solution aqueuse de lavage (24) est de l'eau déminéralisée.

10. Procédé de traitement selon la revendication 1 ou 2, caractérisé en ce que la solution alcaline (16) capable de neutraliser le solvant est de la soude.

11. Procédé de traitement selon l'une des revendications précédentes, caractérisé en ce que le solvant (18) neutralisé sortant de la colonne de réextraction (4) peut être recyclé dans la colonne d'extraction (2).

12. Procédé de traitement selon l'une des revendications précédentes, caractérisé en ce que les étapes d'extraction et de réextraction sont effectuées à température ambiante.

13. Procédé de traitement selon l'une des revendications précédentes, caractérisé en ce que le solvant organique (8) et les autres solutions aqueuses (6, 16, 24) sont mis en contact à contre courant.
